# EUROPÄISCHE PATENTSCHRIFT

(11) **EP 2 339 954 B1**
(45) Veröffentlichungstag und Bekanntmachung des Hinweises auf die Patenterteilung: **24.04.2013**
(21) Anmeldenummer: 09777661.1
(22) Anmeldetag: 05.08.2009
(51) Int. Cl.: A61B 5/00, G06F 19/00

(54) **VERFAHREN ZUM BETREIBEN EINES PATIENTENÜBERWACHUNGSSYSTEMS UND PATIENTENÜBERWACHUNGSSYSTEM**
METHOD FOR OPERATING A PATIENT MONITORING SYSTEM AND PATIENT MONITORING SYSTEM
PROCÉDÉ POUR FAIRE FONCTIONNER UN SYSTÈME DE SURVEILLANCE DE PATIENT ET SYSTÈME DE SURVEILLANCE DE PATIENT

(30) Priorität: 06.08.2008 DE 102008036820
(43) Veröffentlichungstag der Anmeldung: 06.07.2011
(73) Patentinhaber: Hyb D.O.O., 8310 Sentjernej (SI); Beck, Bernd, 72414 Rangendingen (DE)
(72) Erfinder: BECK, Bernd, 72414 Rangendingen (DE); SIMONCIC, Alojz, 8310 Sentjernej (SI); PAVLIN, Marko, 8000 Novo Mesto (SI); GRAMC, Janez, 8263 Cerclje ob Krki (SI)
(74) Vertreter: Bulling, Alexander
(86) Internationale Anmeldenummer: PCT/EP2009/005658
(87) Internationale Veröffentlichungsnummer: WO 2010/015390

(56) Entgegenhaltungen:
- EP-A- 0 602 459
- EP-A- 1 574 164
- DE-U1-202006 014 171
- DE-U1-202006 014 172
- US-A1- 2002 013 517

## Beschreibung

Die Erfindung betrifft ein Verfahren zum Betreiben eines Patientenüberwachungssystems mit einer am Patient abgenommene Messdaten über eine Funkstrecke versendenden Sendeeinheit und mit mehreren Empfangseinheiten zum Empfangen der von der Sendeeinheit ausgesendeten Messdaten. Dabei ist der Sendeeinheit und den Empfangseinheiten jeweils ein eindeutiger Identifikationscode zugeordnet. Eine Funkverbindung zum Übersenden der Messdaten wird nur dann hergestellt, wenn die Sendeeinheit an der jeweiligen Empfangseinheit unter ., Verwendung ihres Identifikationscodes angemeldet wurde. Die Erfindung betrifft ebenfalls ein Patientenüberwachungssystem zur Durchführung eines derartigen Verfahrens.

Die am Patient abgenommene Messdaten versendende Sendeeinheit bewegt sich in der Regel mit dem Patient. In Vorbereitung einer Operation kann diese beispielsweise in einem Vorbereitungsraum patientenseitig angeordnet werden, sich dann mit dem Patient in einen OP-Saal bewegen und nach der Operation mit dem Patient zusammen in den Aufwachraum oder in die Intensivstation verbracht werden. Die mehreren Empfangseinheiten, mit denen die eine Sendeeinheit verbunden sein kann, können beispielsweise an verschiedenen Orten, wie in einem Operationssaal, in einem Vorbereitungsraum, in einem Aufwachraum et cetera vorhanden sein. Die von der Sendeeinheit versendeten abgenommenen Messdaten können dabei entweder als Rohdaten oder auch als verarbeitete beziehungsweise aufbereitete Daten über die Funkstrecke versendet werden.

Ein entsprechendes Verfahren sowie ein zugehöriges Patientenüberwachungssystem ist beispielsweise aus der DE 20 2006 014 171 U1 vorbekannt. Eine Anmeldung zum Aufbau einer Funkverbindung erfolgt bei der DE 20 2006 014 171 U1 durch manuelle Eingabe des Identifikationscodes der Sendeeinheit mittels einer Tastatur an der jeweiligen Empfangseinheit. Es besteht dann eine gesicherte Funkverbindung zu mehreren Empfangseinheiten.

Die Funkverbindung zwischen der Sendeeinheit und den mehreren Empfangseinheiten ist insbesondere bidirektional. Die Begriffe Empfangseinheit und Sendeeinheit sind zum leichteren Verständnis gewählt, um Einheiten voneinander zu unterscheiden und den wesentlichen Betrieb zu charakterisieren, bei dem die patientenseitigen Messdaten von der Sendeeinheit zu der Empfangseinheit übertragen werden. Die Empfangseinheit kann dabei an einen Monitor angeschlossen sein, an dem die abgenommenen Messdaten graphisch angezeigt werden.

Um feststellen zu können, mit welcher beziehungsweise welchen Empfangseinheiten die eine Sendeeinheit in Verbindung steht, ist aus der DE 20 2006 014 171 U1 vorbekannt, eine Testtaste an der Sendeeinheit zu drücken, wodurch in den jeweiligen Empfangseinheiten angezeigt wird, mit welcher beziehungsweise welchen Empfangseinheiten eine Funkverbindung besteht. Die Anzeige erfolgt nur über eine kurze vorbestimmte Zeitdauer von einigen Sekunden und erlischt dann wieder. Denkbar ist auch, dass die Anzeige so lange erfolgt, wie die Testtaste gedrückt wird.

Ähnliche Patientenüberwachungssysteme sind beispielsweise aus der DE 102 21 179 A1 und der DE 10 2004 012 042 A1 bekannt geworden. Bei diesen Systemen ist allerdings stets vorgesehen, dass eine Sendeeinheit nur mit einer Empfangseinheit kommunizieren kann. Eine Kommunikation mit mehreren Empfangseinheiten wird bei diesen Veröffentlichungen gerade nicht gewünscht (vgl. daru beispiels weise Alstz [0040] der

DE 10 2004 012 042 A1, wo ausgegführt wird, dass nach dem ein Verbindungsaufbau ergfolgreich durchgeführt werden ist, ein weiterer Verbindungsaufbau mit anderen Empfängern gesperrt ist). Aus der US 2002/0013517 A1 ist bekannt, an Patientenmonitoren Geräte- oder Verbindungsprobleme anzuzeigen.

Der Vorliegenden Erfindung liegt die Aufgabe zugrunde, ein aus der DE 20 2006 014 171 U1 vorbekanntes Patientenüberwachungssystem mit zugehörigem Verfahren zum Betreiben des Systems derart weiterzubilden, dass die Überwachungssicherheit des Patienten und die Anwenderfreundlichkeit weiter erhöht wird.

Diese Aufgabe wird gelöst durch ein Verfahren mit den Merkmalen des Eatentanspruchs 1. Das Verfahren zeichnet sich folglich dadurch aus, dass an der Sendeeinheit dauerhaft angezeigt wird, ob eine Funkverbindung mit einer beziehungsweise mehreren Empfangseinheiten und/oder mit welcher beziehungsweise welchen Empfangseinheiten besteht. Da jeder Empfangseinheit ein Identifikationscode zugeordnet ist, kann an der Sendeeinheit folglich unter Verwendung des jeweiligen Identifikationscodes angezeigt werden, mit welcher Empfangseinheit eine Funkverbindung besteht. Ferner kann angezeigt werden, ob überhaupt eine Funkverbindung mit einer oder mehreren Empfangseinheiten vorhanden ist. Entscheidenc ist, dass die Anzeige dauerhaft ist und nicht, wie aus der DE 20 2006 014 171 U1 vorbekannt ist, nur von kurzer Zeitdauer beziehungsweise nur wenige Sekunden angezeigt wird. Dadurch, dass die Anzeige an der Sendeeinheit, also patientenseitig erfolgt, kann zudem überwacht werden, ob die patientenseitigen Messdaten überhaupt übermittelt werden. Dadurch, dass angezeigt wird, ob eine Funkverbindung mit einer oder mehreren Empfangseinheiten besteht, kann der Patient beispielsweise aus einem Vorbereitungsraum, in welchem eine Empfangseinheit angeordnet ist, in den Operationssaal überführt werden, ohne dass eine Überwachung der Messdaten unterbrochen wird. Sollte eine Unterbrechung der Funkverbindung auftreten, so kann dies aufgrund der Anzeige an der Sendeeinheit sofort bemerkt werden. Insofern kann mit dem erfindungsgemäßen Verfahren die Überwachungssicherheit erhöht werden.

Ferner ist erfindungsgemäß denkbar, dass nicht nur an der Sendeeinheit, sondern auch an einer Empfangseinheit dauerhaft angezeigt wird, ob eine Funkverbindung mit einer und/oder mit welcher Sendeeinheit besteht. Sollte eine Unterbrechung der Funkverbindung auftreten, so kann dies auch aufgrund der Anzeige an der Empfangseinheit sofort bemerkt werden. Damit ist der Anwender jederzeit in der Lage patientenseitig und/oder empfangseinheitenseitig die jeweilige Funkverbindung eindeutig zu verifizieren.

Eine Weiterbildung der Erfindung sieht vor, dass dann das Vorhandensein einer Funkverbindung an der Sendeeinheit und/oder an einer Empfangseinheit nicht mehr angezeigt wird, wenn eine manuelle Abmeldung der jeweiligen Empfangseinheit erfolgt oder wenn für insbesondere einen vorgebbaren Zeitraum keine Funkverbindung mit der jeweiligen Empfangseinheit mehr besteht. Eine Funkverbindung kann folglich zum einen gezielt abgemeldet werden, beispielsweise durch Betätigen von entsprechenden Resettasten an der Sendeeinheit oder an der Empfangseinheit. Zum anderen ist denkbar, dass eine Funkverbindung abreißen kann, wenn sich der Patient samt Sendeeinheit aus dem Funkbereich entfernt. Bei einem Abriss der Verbindung erfolgt vorzugsweise an der Sendeeinheit und an der jeweiligen Empfangseinheit eine Meldung, dass die Verbindung unterbrochen ist. Sollte der entsprechende Patient samt Sendeeinheit wieder in den Funkbereich der jeweiligen Empfangseinheit eintreten, so kann vorgesehen sein, dass zur Anmeldung der Sendeeinheit an der Empfangseinheit der Identifikationscode der Sendeeinheit an der jeweiligen Empfangseinheit - wie aus der DE 20 2006 014 171 U1 und der DE 10 2004 012042 A1 vorbekannt ist - erneut manuell eingegeben werden muss.

Gemäß einer weiteren vorteilhaften Ausgestaltung der Erfindung ist vorgesehen, dass zum Aufbau der Funkverbindung die Sendeeinheit dann, wenn wenigstens eine beziehungsweise mehrere Empfangseinheiten im Funkbereich der Sendeeinheit vorhanden ist beziehungsweise sind, unter Verwendung ihres Identifikationscodes eine Anmeldeabfrage an die jeweilige Empfangseinheit sendet und eine Anmeldung der Sendeeinheit an der jeweiligen Empfangseinheit und damit der Aufbau einer Funkverbindung für die Übersendung der Messdaten nur dann erfolgt, wenn die Anmeldeabfrage an der Empfangseinheit insbesondere manuell bestätigt wird. Die Anmeldung der Sendeeinheit an der jeweiligen Empfangseinheit erfolgt also durch eine Bestätigung des von der Sendeeinheit empfangenen Identifikationscodes an der Empfangseinheit. Anders als beim vorbekannten Stand der Technik in Form der DE 20 2006 014 171 U1 oder der DE 10 2004 012 042 A1 ist folglich vorgesehen, dass der Identifikationscode der jeweiligen Sendeeinheit nicht manuell mittels einer Tastatur an der jeweiligen Empfangseinheit einzugeben ist. Vielmehr übersendet die Sendeeinheit ihren Identifikationscode an die im Funkbereich vorhandenen Empfangseinheiten. Dieser wird dort insbesondere an einem Display angezeigt. Zum Aufbau der Funkverbindung muss dann lediglich die Anmeldeabfrage der Sendeeinheit beziehungsweise der an der Empfangseinheit angezeigte Identifikationscode der Sendeeinheit bestätigt werden, beispielsweise durch Auswählen der jeweiligen Sendeeinheit - falls mehrere Sendeeinheiten im Funkbereich der Empfangseinheit sind - und durch Drücken einer entsprechenden Taste beziehungsweise Berühren eines entsprechenden Anzeigefeldes im Display der Empfangseinheit. Sollten im Funkbereich der Empfangseinheit mehrere unterschiedliche Sendeeinheiten vorhanden sein, so werden Anmeldeabfragen an die jeweilige Empfangseinheit gesendet und an der Empfangseinheit werden dann sämtliche im Funkbereich vorhandene Sendeeinheiten, insbesondere unter Verwendung ihres Identifikationscodes, angezeigt. Da allerdings eine Empfangseinheit insbesondere nur mit einer Sendeeinheit für die Übersendung der Messdaten eine Funkverbindung herstellen kann, ist folglich an der Empfangseinheit auszuwählen, mit welcher Sendeeinheit letztlich eine Funkverbindung hergestellt werden soll. Diese Auswahl ist dann manuell, beispielsweise mittels eines Cursors im Bedienfeld der Empfangseinheit, vorzunehmen und zu bestätigen.

Wie bereits erwähnt, ist dabei vorteilhaft, wenn an der jeweiligen Empfangseinheit angezeigt wird, ob und von welcher Sendeeinheit eine Anmeldeabfrage vorliegt. Folglich wird angezeigt, welche Sendeeinheiten sich im Funkbereich der Empfangseinheit befinden.

Vorteilhaft ist, wie ebenfalls bereits erwähnt, wenn aufgrund der Anmeldeabfrage der Identifikationscode der Sendeeinheit an der jeweiligen Empfängereinheit angezeigt wird und dass durch manuelles Auswählen und Bestätigen der Auswahl eine Anmeldung und damit ein Aufbau der die Messdaten übertragenden Funkverbindungen erfolgt, ohne dass der komplette Identifikationscode, wie es beim vorbekannten Stand der Technik der Fall ist, manuell über eine Tastatur eingegeben werden muss. Manuelle Bestätigung heißt folglich bei dieser Weiterbildung der Erfindung lediglich Auswählen des jeweiligen Identifikationscodes und Bestätigen der Auswahl.

Ferner ist vorteilhaft, wenn die Sendeeinheit dann ein Signal abgibt, wenn für eine vorgebbare Zeitdauer keine Funkverbindung zu wenigstens einer Empfangseinheit besteht. Hierdurch wird die Aufmerksamkeit darauf gelenkt, dass eine Überwachung des Patienten nicht gegeben ist. Eine weitere bevorzugte Ausführungsform der Erfindung sieht vor, dass nach einer Unterbrechung der Funkverbindung zwischen der Sendeeinheit und einer Empfangseinheit eine Funkverbindung auch noch nach einem längeren Zeitraum, der im Bereich wenigen Minuten liegen kann, zwischen der Sendeeinheit und der Empfangseinheit automatisch wieder hergestellt wird, ohne dass eine erneute Anmeldung erforderlich ist oder der Identifikationscode der Sendeeinheit nochmals an der Empfangseinheit einzugeben oder zu bestätigen ist. Sollte also der entsprechende Patient samt Sendeeinheit nach Verlassen des Funkbereichs oder nach einer Unterbrechung der Verbindung wieder in den Funkbereich der jeweiligen Empfangseinheit eintreten, so wird die Verbindung reaktiviert und wiederaufgenommen. Entgegen dem Stand der Technik, wie aus der DE 20 2006 014 171 U1 und der DE 10 2004 012042 A1 vorbekannt ist, wird der Identifikationscode der Sendeeinheit im Datenspeicher der Empfangseinheit bei Verbindungsabbruch nicht gelöscht, sondern bleibt dort gespeichert. Dadurch kann - im Gegensatz zur DE 10 2004 012 042 A1, bei der gerade vorgesehen ist, dass eine Funkverbindung nach einer Unterbrechung, die länger als eine vorgegebene Zeitdauer dauert, nicht wieder automatisch hergestellt wird - die Funkverbindung wieder automatisch aufgenommen werden. Folglich werden beim Wiedereintritt in den Funkbereich die Messdaten sofort wieder übertragen, ohne dass eine erneute Anmeldung der Sendeeinheit an der Empfangseinheit zu erfolgen hat. Dies führt zu einer vereinfachten und benutzfreundlichen Handhabung des Überwachungssystems.

Die jeweils gegenseitig kommunizierenden Einheiten werden dauerhaft unter Verwendung des jeweiligen Identifikationscodes an der jeweiligen Anzeige angezeigt. Der Funkpartner ist also sofort wieder eindeutig verifizierbar und eine Verwechslung mit einem anderen Patienten, beziehungsweise anderen Sendeeinheit kann somit ausgeschlossen werden.

Besonders vorteilhaft ist hierbei, wenn an der Sendeeinheit und/oder an der Empfangseinheit eine Wiederherstellung der Funkverbindung angezeigt wird. Die Anzeige kann insbesondere über eine Meldung am Display erfolgen, insbesondere unter Angabe des Identifikationscodes der jeweiligen Einheit. Der jeweilige Benutzer erhält folglich Kenntnis, dass die Verbindung wieder besteht. Insbesondere kann dabei nochmals klarstellend angegeben werden, zu welcher Einheit die Verbindung besteht.

Ferner ist denkbar, dass der Benutzer angefragt wird, ob er die bestehende Verbindung weiter aufrecht erhalten möchte oder ob die bestehende Verbindung abgebrochen bzw. abgemeldet werden soll.

Eine weitere, besonders bevorzugte Ausführungsform der Erfindung sieht vor, dass an der Sendeeinheit dem Identifikationscode der Sendeeinheit ein Benutzer definiertes Identifikationswort und/oder dass an der Empfangseinheit dem Identifikationscode der Empfangseinheit ein Benutzer definiertes Identifikationswort zuweisbar ist, das anstelle des jeweiligen Identifikationscodes Verwendung findet. Hierdurch wird vorteilhafterweise erreicht, dass beispielsweise dem Identifikationscode einer Sendeeinheit der Name des jeweiligen Patienten zugeordnet werden kann und/oder ein anderer, frei wählbarer Codename. Folglich wird dann an der Empfangseinheit, die mit der Sendeeinheit kommuniziert, insbesondere nicht nur der Identifikationscode der Sendeeinheit, sondern auch das Identifikationswort bzw. der Name des jeweiligen Patienten oder ein anderer Codename angezeigt. Denkbar ist auch, dass lediglich das Identifikationswort angezeigt wird. Hierdurch wird insgesamt auch die Überwachungssicherheit sowie die Anwenderfreundlichkeit erhöht, da die Zuordnung von Sendeeinheiten zu Empfangseinheiten vereinfacht, beschleunigt und verdeutlicht wird. Die Empfangseinheiten können ebenfalls ein passendes Identifikationswort zugeordnet bekommen. Einer Empfangseinheit in einem Operationssaal 1 kann beispielsweise das Identifikationswort OP1 zugeordnet werden.

Ferner ist vorteilhaft, wenn in der Sendeeinheit und/oder den jeweiligen Empfangseinheiten gespeichert wird, wann und wie lange eine Funkverbindung mit welcher Empfangseinheit und/oder Sendeeinheit bestand. Durch eine derartige Speicherung kann die Historie der Überwachung nachvollzogen werden und besondere Ereignisse können abgefragt werden. Beispielsweise kann nachvollzogen werden, ob der Patient im Falle einer Operation kontinuierlich und dauerhaft vom Vorbereitungsraum über den Operationssaal bis hin zum Aufwachraum überwacht wurde.

Die eingangs genannte Aufgabe wird auch gelöst mittels eines Patientenüberwachungssystems mit einer am Patient abgenommene Messdaten über eine Funkstrecke versendenden Sendeeinheit und mit mehreren Empfangseinheiten zum Empfangen der von der Sendeeinheit ausgesendeten Messdaten, wobei an der Sendeeinheit Anzeigemittel zur dauerhaften Anzeige, ob eine Funkverbindung zwischen der Sendeeinheit und einer Empfangseinheit besteht und/oder mit welcher Empfangseinheit eine Funkverbindung besteht. Dabei ist vorteilhaft, wenn die Anzeigemittel ein Display und/oder eine Leuchtanzeige umfassen.

Ein solches System kann insbesondere zudem vorsehen, dass an der Empfangseinheit Anzeigemittel vorgesehen sind zur dauerhaften Anzeige, ob eine Funkverbindung mit der Sendeeinheit besteht.

Insbesondere ist vorteilhaft, wenn wenigstens eine Empfangseinheit einen Monitor zur Anzeige der Messwerte umfasst. Allerdings ist auch denkbar, dass eine Empfangseinheit Schnittstellen zum Anschluss eines Monitors aufweist. Vorteilhaft ist in jedem Falle, wenn die Empfangseinheiten sowie die zugehörige Sendeeinheit ein Display zum Anzeigen entsprechender Informationen, wie Identifikationscodes oder Identifikationsworte, Anwenderempfehlungen, Instruktionen, Abfragen sowie Gerätezustandsmeldungen, z.B. den Batterieladezustand, Funksignalstärke und andere Zustandsmeldungen aufweisen. Ferner ist vorteilhaft, wenn die Sendeeinheit, insbesondere zusätzlich zu einem Netzanschluss, eine auswechselbare Batterie zur Versorgung der Sendeeinheit mit Energie aufweist und wenn die Sendeeinheit zusätzlich eine interne Batterie aufweist, die die Sendeeinheit dann mit Energie versorgt, wenn die auswechselbare Batterie entfernt wird. Eine Entfernung der auswechselbaren Batterie erfolgt insbesondere dann, wenn die Batterie leer ist. Mittels der internen Batterie kann dann die Sendeeinheit mit ausreichender Energie, zumindest für wenige Minuten, versorgt werden.

Bei der auswechselbaren Batterie handelt es sich vorzugsweise um eine wiederaufladbare Batterie. Vorteilhaft ist dann, wenn an der Empfangseinheit eine Ladestation zum Laden der auswechselbaren Batterie der Sendeeinheit vorgesehen ist. Insbesondere können mehrere auswechselbare Batterien in Umlauf sein, wobei sich eine in der Sendeeinheit befindet und weitere Batterien in der jeweiligen Ladestation der jeweiligen Empfangseinheit. Hierdurch wird gewährleistet, dass stets eine aufgeladene Batterie griffbereit ist.

Weitere Einzelheiten und vorteilhafte Ausgestaltungen der Erfindung sind der nachfolgenden Beschreibung zu entnehmen, anhand derer die Erfindung näher beschrieben und erläutert wird.

Es zeigen:
- Figur 1: ein erfindungsgemäßes Patientenüberwachungssystem nach dem Betätigen der On-Taste;
- Figur 2: eine Empfangseinheit des Systems nach Figur 1 im Rename-Modus;
- Figur 3: System gemäß Figur 1 im Suchmodus;

- Figur 4: das System gemäß Figur 1 im Abfragemodus;
- Figur 5: das System gemäß Figur 1 bei aufgebauter Funkverbindung; und
- Figur 6: das System gemäß Figur 5 bei abgerissener Funkverbindung.

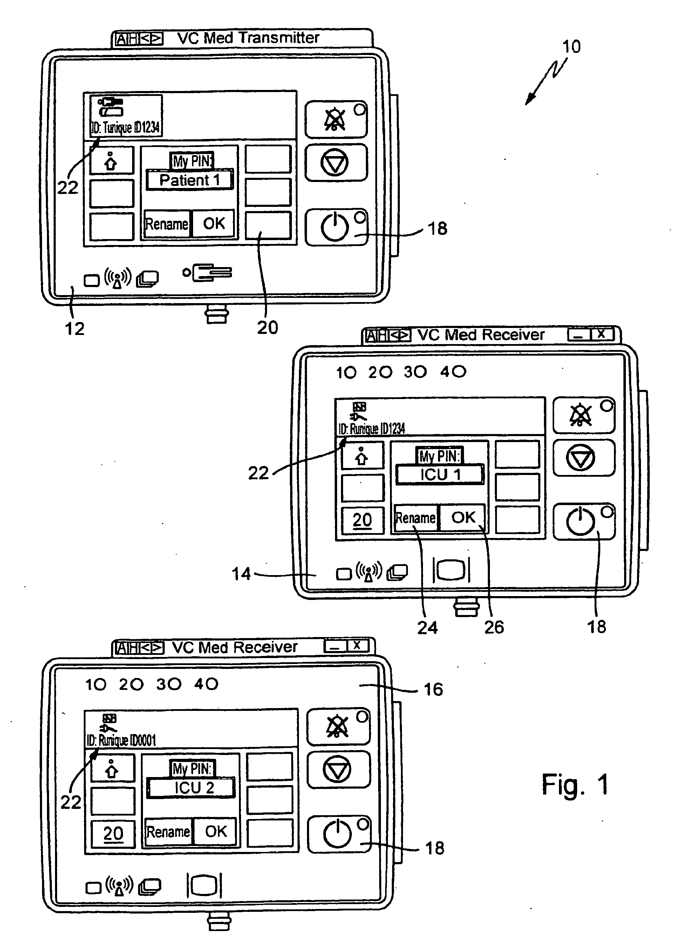

In der Figur 1 ist ein erfindungsgemäßes Patientenüberwachungssystem 10 angedeutet, das eine Sendeeinheit 12 und zwei Empfangseinheiten 14 und 16 umfasst. Mit der Sendeeinheit 12 können an einem Patienten abgenommene Messdaten, wie beispielsweise Blutdruck, EKG, Sauerstoffpartialdruck, invasiver Blutdruck oder dergleichen an die Empfänger 14 und 16, und falls weitere Empfänger vorhanden sind, an diese weiteren Empfänger versendet werden. Dazu ist die Sendeeinheit 12 insbesondere über eine Kabelverbindung mit entsprechenden Messaufnehmern oder Transducern verbunden. Eine Integration in einem Gerät ist auch denkbar. In Betrieb verbleibt die Sendeeinheit 12 folglich beim Patienten, da an diesem die Messdaten abgenommen werden. Insbesondere vor, während und nach einer Operation sowie im Intensivüberwachungsbereich werden erwähnte Messdaten am Patienten erfasst und überwacht.

Zur Überwachung der Messdaten werden diese, wie erwähnt, von der Sendeeinheit 12 zu den Empfangseinheiten 14, 16 versendet. Die Empfangseinheiten 14, 16 sind dabei insbesondere an einen Monitor anschließbar, wo die entsprechenden Messdaten in der Regel graphisch dargestellt werden. Allerdings ist auch denkbar, dass die Empfangseinheiten 14, 16 einen Monitor aufweisen, an dem die empfangenen Messdaten angezeigt werden. Die Messdaten können dabei von der Sendeeinheit 12 vor dem Versenden verarbeitet beziehungsweise aufbereitet werden. Andererseits ist denkbar, dass die abgenommenen Rohdaten an die Empfangseinheiten 14, 16 versendet werden.

Die Versendung der Signale erfolgt insbesondere im Bluetooth-Standard. Andere Funkverbindungen, insbesondere im GH_{z} Bereich, sind auch denkbar.

Die Sendeeinheit 12 sowie die Empfangseinheiten 14, 16 umfassen jeweils eine Einschalttaste 18 zur Inbetriebnahme der jeweiligen Einheit. Die Einheiten 12, 14, 16 umfassen zudem einen als Touchscreen ausgebildeten Monitor 20. Nach dem Einschalten der Einheiten 12, 14, 16 werden die in Figur 1 dargestellten Oberflächen an den jeweiligen Touchscreens 20 gezeigt. An der Sendeeinheit 12 wird ein der Sendeeinheit 12 zugeordnetes Identifikationswort (My PIN) angezeigt, das in Figur 1 Patient1 heißt. Der Sendeeinheit 12 ist außerdem ein eindeutiger Identifikationscode ID zugeordnet: Tunique ID 0001. Dieser Identifikationscode wird im Bereich 22 angezeigt. Das Identifikationswort Patient1 wird folglich diesem ID zugeordnet.

Entsprechend weist die Empfangseinheit einen eindeutigen Identifikationscode auf, nämlich ID: Runique ID 1234, dem das Identifikationswort ICU1 zugeordnet ist. Entsprechend ist dem eindeutigen Identifikationscode der Einheit 16 ID: Runique ID 0001 das Identifikationswort ICU2 zugeordnet.

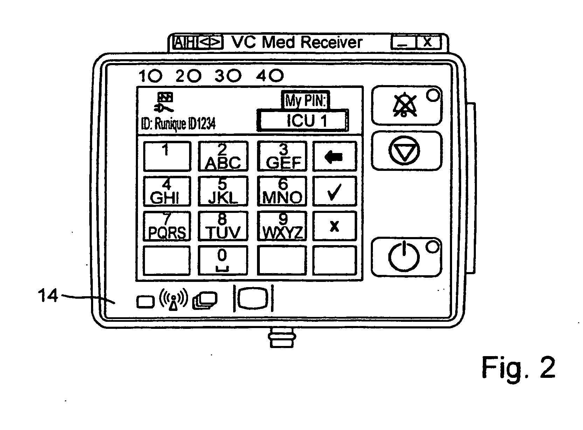

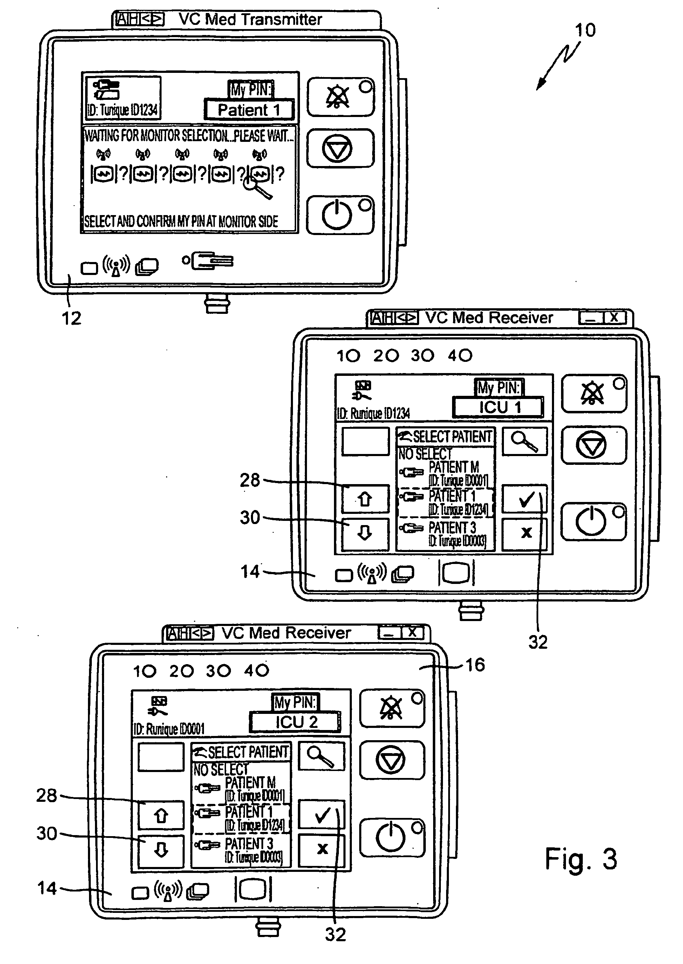

Durch Drücken der Rename-Taste 24 kann dem jeweiligen Identifikationscode ein beliebiges Identifikationswort zugeordnet werden. Figur 2 zeigt den Rename-Modus, in dem durch Drücken der entsprechenden Tasten das Identifikationswort ICU1 in ein beliebiges anderes Identifikationswort, beispielsweise OP1, geändert werden kann. Entsprechend können die Identifikationsworte Patient1 und ICU2 durch Drücken der jeweiligen Rename-Taste 24 geändert werden. Wird das Identifikationswort beziehungsweise My PIN als korrekt angesehen, so kann die OK-Taste 26 betätigt werden. Die Touchscreens 20 wechseln dann in den in Figur 3 gezeigten Modus. Die Sendeeinheit 12 sendet in diesem Modus eine Anmeldeabfrage an die Empfangseinheiten 14, 16 und wartet auf eine empfangseinheitenseitige Bestätigung zur Anmeldung der Sendeeinheit 12 und damit zum Aufbau einer Funkverbindung. An den Empfangseinheiten 14 und 16 wird angezeigt, welche Sendeeinheiten 12 im Funkbereich vorhanden sind und eine entsprechende Anmeldeabfrage versendet haben. Auf dem Touchscreen der Empfangseinheiten 14 und 16 werden beispielhaft jeweils drei Sendeeinheiten angezeigt, die im Funkbereich vorhanden sind, und mit denen eine Funkverbindung hergestellt werden könnte. Dazu wird von den Sendeeinheiten jeweils das frei gewählte Codewort sowie dahinter, in Klammern, der zugehörige Identifikationscode angezeigt. Die im Funkbereich vorhandenen Sendeeinheiten senden folglich ihren Identifikationscode samt Identifikationswort an die Empfangseinheiten 14, 16.

Zum Aufbau einer die Messdaten übertragenden Funkverbindung ist die gewünschte Sendeeinheit empfängerseitig zu bestätigen. Das erfolgt durch Auswahl der jeweiligen Sendeeinheit durch Drücken der Auswahltasten 28, 30. Ist die gewünschte Sendeeinheit ausgewählt, so wird dies durch Drücken der Bestätigungstaste 32 bestätigt.

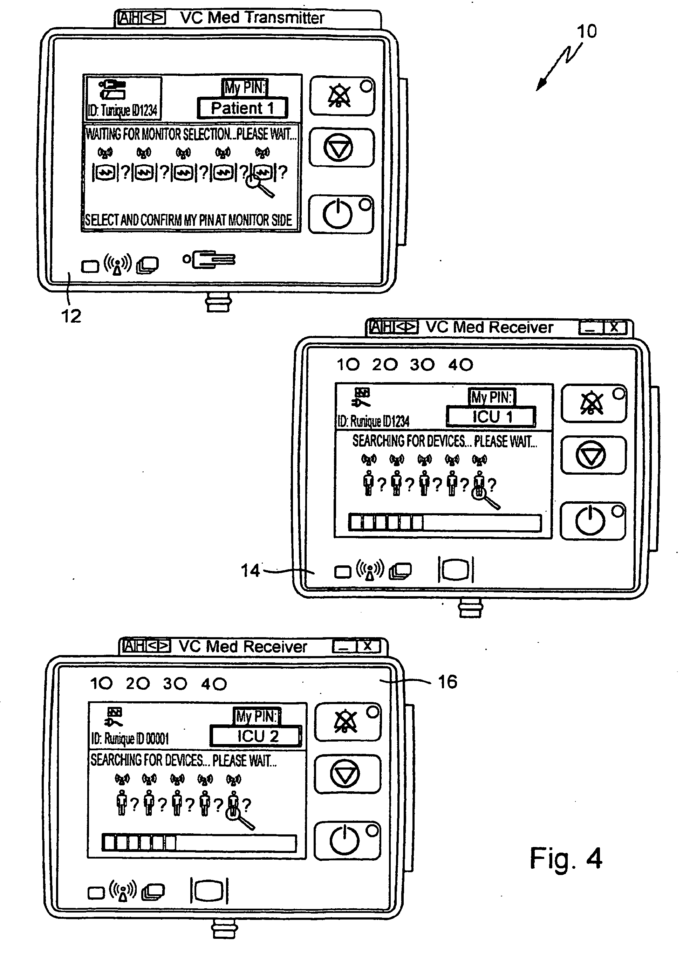

Nachdem die jeweilige Sendeeinheit ausgewählt wurde und die Auswahl bestätigt wurde, erfolgt der Aufbau der Funkverbindung zum Austausch der Messdaten. Figur 4 zeigt die Anzeige des jeweiligen Touchscreens während des Verbindungsaufbaus.

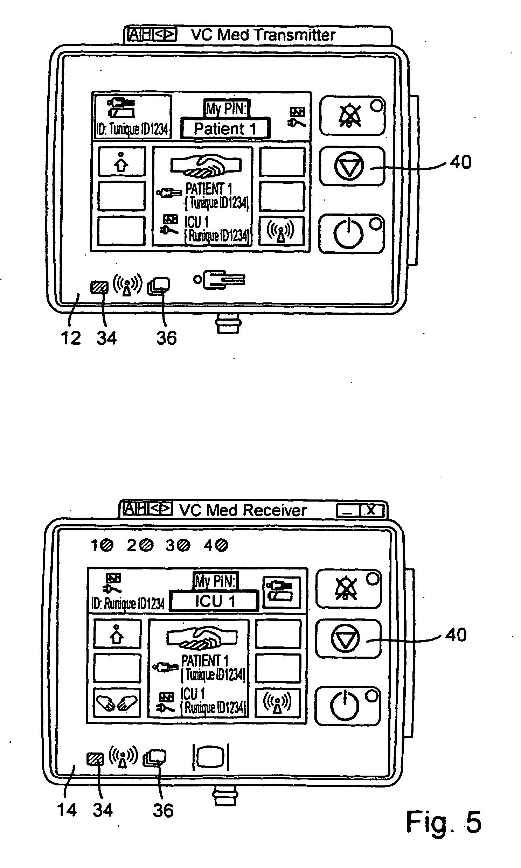

In Figur 5 ist der Betriebsmodus nach erfolgtem Erfindungsaufbau dargestellt; das sogenannte Pairing wurde erfolgreich abgeschlossen.

Wie dem Touchscreen 20 der Sendeeinheit 12 in Figur 5 entnommen werden kann, ist die Sendeeinheit 12, das heißt Patient1, verbunden mit der Empfangseinheit 14, das heißt mit ICU1. Dies wird am Touchscreen 20 der Sendeeinheit dauerhaft angezeigt. In Figur 5 ist folglich lediglich eine Verbindung zwischen der Sendeeinheit 12 und der Empfangseinheit 14 hergestellt; eine Verbindung zur Empfangseinheit 16 besteht gemäß Figur 5 nicht.

Die Sendeeinheit 12 sowie die Empfangseinheiten 14 und 16 umfassen separat vom Touchscreen ausgebildete Anzeigen 34 und 36, mit denen angezeigt wird, ob lediglich mit einer weiteren Einheit eine Verbindung besteht oder ob mit mehreren weiteren Einheiten eine Verbindung besteht. Besteht lediglich eine Verbindung mit einer Einheit, so leuchtet die Anzeige 34 auf. Besteht eine Verbindung mit mehreren weiteren Einheiten, so leuchtet die Anzeige 36 auf. Da in Figur 5 eine Verbindung der Sendeeinheit 12 mit lediglich einer Empfangseinheit 14 besteht, leuchtet folglich die Anzeige 34 auf. Für den Fall, dass eine weitere Verbindung mit der Sendeeinheit 12 bestehen würde, würde dann die Anzeige 34 erlöschen und die Anzeige 36 an der Sendeeinheit 12 aufleuchten. Zudem würde das Identifikationswort samt Identifikationscode der weiteren Empfangseinheit 16 am Display 20 der Sendeeinheit 12 angezeigt werden.

Befindet sich der Patient beispielsweise in Vorbereitung einer Operation in einem Vorbereitungsraum, in dem ein Empfänger vorhanden ist, so wird dieser Empfänger bei bestehender Funkverbindung sendeseitig dauerhaft angezeigt. Sollte bereits im Vorbereitungsraum eine Verbindung zu einem im Operationssaal vorhandenen Empfänger bestehen, so würde dieses ebenfalls senderseitig angezeigt. Sollte ferner in einem Aufwachraum eine weitere Empfangseinheit vorhanden sein, so würde dies, falls eine Funkverbindung besteht, ebenfalls senderseitig angezeigt werden. Folglich kann stets senderseitig festgestellt werden, ob und mit welchen Empfangseinheiten eine Verbindung vorhanden ist. Andererseits kann an der jeweiligen Empfangseinheit festgestellt werden, zu welchem Patienten eine Verbindung vorhanden ist und von welchem Patienten entsprechende Messdaten graphisch angezeigt werden.

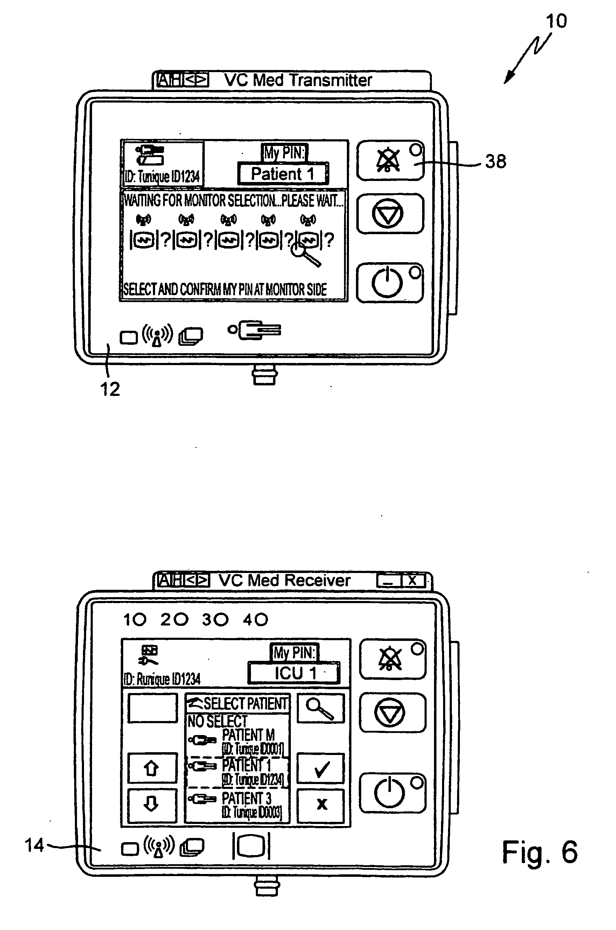

Verlässt die Sendeeinheit 12 den Funkbereich der Empfangseinheit 14, so wird dies an der Sendeeinheit 12 als auch an der Empfangseinheit 14 dargestellt, wie in Figur 6 gezeigt. Da keine Funkverbindung mehr besteht, fällt die Sendeeinheit 12 zurück in den Abfragemodus und sucht im Funkbereich vorhandene Empfangseinheiten. Entsprechend wird an der Empfangseinheit 14 angezeigt, welche Sendeeinheiten im Funkbereich noch vorhanden sind. Zum Wiederherstellen einer Verbindung ist die jeweilige Sendeeinheit beziehungsweise der zugehörige Patient neu anzumelden, d.h. auszuwählen und die Auswahl ist zu bestätigen.

Insbesondere kann vorgesehen sein, dass die Verbindung automatisch ohne das Vornehmen einer Neuanmeldung wieder hergestellt wird, sobald diejenige Sendeeinheit, die zuvor den Empfangsbereich des zugehörigen Empfängers verlassen hatte, wieder in den Empfangsbereich eintritt. Messdaten werden folglich dann automatisch wieder übertragen und am Monitor angezeigt. Der Identifikationscode der Sendeeinheit wird dazu im zugehörigen Datenspeicher des Empfängers nicht gelöscht.

Dabei kann ergänzend ein Warnhinweis mittels eines optischen und/oder akustischen Signals an der Empfangseinheit ausgegeben werden, verbunden mit einer Anzeige auf dem Display 20 der Empfangseinheit 14, 16, dass die zuvor über einen Zeitraum hinweg abgerissene Funkverbindung wieder aufgenommen wurde.

Mit der Wiederherstellung der Funkverbindung wird der Benutzer insbesondere an der Empfangseinheit angefragt, ob die bestehende Funkverbindung weiter aufrecht erhalten bleiben soll oder ob sie abgebrochen werden soll. Der Benutzer wird folglich vor die Wahl gesellt, die Verbindung aufrecht zu erhalten oder abzubrechen. Der jeweilige Funkpartner der Sende- und Empfangseinheit, beziehungsweise deren Identifikationscode und/oder Identifikationswort kann vor, während und nach dieser Anfrage dauerhaft an der jeweiligen Sendeeinheit sowie an der einen oder den mehreren Empfangseinheit/en angezeigt bleiben. Nach Beantworten der Anfrage erlöscht diese; sie kann aber in einem Historie-Menu der Empfangs- und Sendeeinheit über einen zu definierenden Zeitraum gespeichert bleiben.

Um die Funkverbindung manuell zu unterbrechen beziehungsweise die jeweilige Einheit zurückzusetzen, ist an den Einheiten eine Resettaste 40 vorgesehen.

Mit den Anzeigen 34 und 36 wird folglich dauerhaft angezeigt, ob eine Funkverbindung zwischen einer oder mehreren Empfangseinheiten 14, 16 vorgesehen ist. Ferner wird bei vorhandener Funkverbindung auf dem Touchscreen der Sendeeinheit 12 angezeigt, mit welcher beziehungsweise mit welchen Empfangseinheiten 14, 16 eine Funkverbindung besteht. Auf Seiten der Empfangseinheiten 14, 16 wird an dem jeweiligen Touchscreen angezeigt, mit welcher Sendeeinheit 12 eine Funkverbindung vorhanden ist.

Die Sendeeinheit 12 wird vorzugsweise mit einer auswechselbaren Batterie betrieben. Um sicherzustellen, dass beim Auswechselvorgang der Batterie die Sendeeinheit 12 mit ausreichend Strom versorgt wird, ist eine weitere interne Batterie vorgesehen und so ausgelegt, dass sie die Sendeeinheit 12 ausreichend lange mit Strom versorgen kann, bis der Batteriewechsel vollzogen ist, zum Beispiel ein bis zwei Minuten. Nach dem Batteriewechsel kann die interne Batterie, insbesondere über die auswechselbare Batterie oder mittels einer externen Stromversorgung, wieder aufgeladen werden. Die auswechselbare Batterie ist vorzugsweise eine wiederladbare Batterie. Zum Laden der Batterie kann insbesondere an den Empfangseinheiten 14, 16 eine Ladestation vorgesehen sein, so dass stets eine geladene Batterie zur Verfügung steht. Die Empfangseinheiten 14, 16 selbst werden vorzugsweise mit Energie aus einem Stromnetz betrieben, da die Empfangseinheiten regelmäßig einen festen Standort im Bereich eines Monitors haben, wo eine externe Stromversorgung gewährleistet ist.

Anderseits ist denkbar, dass die Empfangseinheiten 14, 16 ebenfalls mobil ausgebildet sein können. In diesem Falle weisen die Empfangseinheiten, entsprechend den Sendeeinheiten, vorzugsweise eine auswechselbare sowie eine interne Batterie auf.

## Patentansprüche

1. Verfahren zum Betreiben eines Patientenüberwachungssystems (10) mit einer am Patient abgenommene Messdaten über eine Funkstrecke versendenden Sendeeinheit (12) und mit mehreren Empfangseinheiten (14, 16) zum Empfangen der von der Sendeeinheit (12) ausgesendeten Messdaten, wobei der Sendeeinheit (12) und den Empfangseinheiten (14, 16) jeweils ein eindeutiger Identifikationscode (ID) zugeordnet ist und eine Funkverbindung zum Übersenden der Messdaten von der einen Sendeeinheit (12) zu den mehreren Empfangseinheiten (14, 16) nur dann hergestellt werden kann, wenn die Sendeeinheit (12) an der jeweiligen Empfangseinheit (14, 16) unter Verwendung ihres Identifikationscodes (ID) angemeldet wurde, und dass eine Messdaten übersendende Funkverbindung zwischen der Sendeeinheit (12) und mehreren Empfangseinheiten (14, 16) bestehen kann, **dadurch gekennzeichnet, dass** an der Sendeeinheit (12) dauerhaft angezeigt wird, mit welcher oder welchen der Empfangseinheiten (14, 16) eine Messdaten übersendende Funkverbindung besteht.

2. Verfahren nach Anspruch 1, **dadurch gekennzeichnet, dass** nicht nur an der Sendeeinheit (12), sondern auch an der Empfangseinheit (14, 16) dauerhaft angezeigt wird, ob eine Funkverbindung mit einer und/oder mit welcher Sendeeinheit (12) besteht.

3. Verfahren nach Anspruch 1 oder 2, **dadurch gekennzeichnet, dass** dann das Vorhandensein einer Funkverbindung an der Sendeeinheit (12) und/oder Empfangseinheit (14, 16) nicht mehr angezeigt wird, wenn eine manuelle Abmeldung der jeweiligen Empfangseinheit (14, 16) erfolgt oder wenn keine Funkverbindung mit der jeweiligen Empfangseinheit (14, 16) mehr besteht.

4. Verfahren nach Anspruch 1, 2 oder 3, **dadurch gekennzeichnet, dass** zum Aufbau der Funkverbindung die Sendeeinheit (12) dann, wenn wenigstens eine der Empfangseinheiten (14, 16) im Funkbereich der Sendeeinheit (12) vorhanden ist, unter Verwendung ihres Identifikationscodes (ID) eine Anmeldeabfrage an die jeweilige Empfangseinheit (14, 16) richtet und eine Anmeldung der Sendeeinheit (12) an der jeweiligen Empfangseinheit (14, 16) und der Aufbau einer Funkverbindung für die Übersendung der Messdaten nur dann erfolgt, wenn die Anmeldeabfrage an der Empfangseinheit (14, 16) bestätigt wird.

5. Verfahren nach Anspruch 4, **dadurch gekennzeichnet, dass** an der jeweiligen Empfangseinheit (14, 16) angezeigt wird, ob und von welcher Sendeeinheit (12) eine Anmeldeabfrage vorliegt.

6. Verfahren nach Anspruch 4 oder 5, **dadurch gekennzeichnet, dass** aufgrund der Anmeldeabfrage der Identifikationscode (ID) der Sendeeinheit (12) an der jeweiligen Empfängereinheit (14, 16) angezeigt wird und dass durch Auswählen und Bestätigen der Auswahl eine Anmeldung erfolgt, ohne dass der Identifikationscode (ID) als solcher manuell über insbesondere eine Tastatur eingegeben werden muss.

7. Verfahren nach wenigstens einem der vorhergehenden Ansprüche, **dadurch gekennzeichnet, dass** die Sendeeinheit (12) dann ein Signal abgibt, wenn für eine vorgebbare Zeitdauer keine Funkverbindung zu wenigstens einer Empfangseinheit (14, 16) mehr besteht.

8. Verfahren nach wenigstens einem der vorhergehenden Ansprüche, **dadurch gekennzeichnet, dass** nach einer sich auch über einen Zeitraum von wenigen Minuten erstreckenden Unterbrechung der Funkverbindung zwischen der Sendeeinheit und einer Empfangseinheit eine Funkverbindung automatisch wieder zwischen der Sendeeinheit und der Empfangseinheit wieder hergestellt wird, ohne dass der Identifikationscode der Sendeeinheit nochmals an der Empfangseinheit anzumelden ist.

9. Verfahren nach Anspruch 8, **dadurch gekennzeichnet, dass** an der Sendeeinheit und/oder an der Empfangseinheit eine Wiederherstellung der Funkverbindung angezeigt wird.

10. Verfahren nach Anspruch 8 oder 9, **dadurch gekennzeichnet, dass** mit oder nach einer Wiederherstellung der Funkverbindung eine Anfrage erfolgt, ob die bestehende Funkverbindung weiter aufrecht erhalten bleiben soll oder ob die bestehende Funkverbindung abgebrochen werden soll.

11. Verfahren nach wenigstens einem der vorhergehenden Ansprüche, **dadurch gekennzeichnet, dass** an der Sendeeinheit (12) dem Identifikationscode (ID) der Sendeeinheit (12) ein benutzerdefiniertes Identifikationswort (PIN) und/oder dass an der Empfangseinheit (14, 16) dem Identifikationscode (ID) der Empfangseinheit ein benutzerdefiniertes Identifikationswort (PIN) zuweisbar ist, das anstelle des jeweiligen Identifikationscodes (ID) Verwendung findet.

12. Verfahren nach wenigstens einem der vorhergehenden Ansprüche, **dadurch gekennzeichnet, dass** in der Sendeeinheit (12) und/oder den jeweiligen Empfangseinheiten (14, 16) gespeichert wird, wann und wie lange eine Funkverbindung mit welcher Empfangseinheit (14, 16) und/oder Sendeeinheit (12) bestand.

13. Patientenüberwachungssystem (10) mit einer am Patient abgenommene Messdaten über eine Funkstrecke versendenden Sendeeinheit (12) und mit mehreren Empfangseinheiten (14, 16) zum Empfangen der von der Sendeeinheit ausgesendeten Messdaten zur Durchführung des Verfahrens nach wenigstens einem der vorhergehenden Ansprüche aufweist, wobei an der Sendeeinheit (12) Anzeigemittel (20, 34, 36) vorgesehen sind zur dauerhaften Anzeige, mit welcher der Empfangseinheiten (14, 16) eine Funkverbindung besteht.

14. System nach Anspruch 13, **dadurch gekennzeichnet, dass** an der Empfangseinheit (14, 16) Anzeigemittel (20, 34, 36) vorgesehen sind zur dauerhaften Anzeige, ob eine Funkverbindung mit der Sendeeinheit (12) besteht.

15. System nach wenigstens einem der Ansprüche 13 oder 14, **dadurch gekennzeichnet, dass** die Sendeeinheit (12) bzw. die Empfangseinheit (14, 16) eine auswechselbare Batterie zur Versorgung der Sendeeinheit mit Energie aufweist und dass die Sendeeinheit (12) bzw. die Empfangseinheit (14, 16) eine interne Batterie umfasst, die die Sendeeinheit bzw. die Empfangseinheit (14, 16) dann mit Energie versorgt, wenn die auswechselbare Batterie entfernt wird.

16. System nach Anspruch 15, **dadurch gekennzeichnet, dass** an der Empfangseinheit (14, 16) bzw. der Sendeeinheit (12) eine Ladestation zum Laden der auswechselbaren Batterie der Sendeeinheit (12) bzw. der Empfangseinheit (14, 16) vorgesehen ist.

## Claims

1. Method for operating a patient monitoring system (10) with a transmitter unit (12) emitting via a radio link measurement data recorded on the patient, and with multiple receiver units (14, 16) for receiving the measurement data transmitted by the transmitter unit (12), wherein a unique identification code (ID) is allocated to the transmitter unit (12) and each receiver unit (14, 16) respectively, and a radio connection for transmission of measurement data from the one transmitter unit (12) to the multiple receiver units (14, 16) can only be created if the transmitter unit (12) has logged onto the respective receiver unit (14, 16) using its identification code (ID), and that a radio connection transmitting measurement data can exist between the transmitter unit (12) and multiple receiver units (14, 16), **characterised in that** the transmitter unit (12) permanently displays with which receiver unit or units (14, 16) a radio connection transmitting measurement data exists.

2. Method according to claim 1, **characterised in that** not only the transmitter unit (12) but also the receiver unit (14, 16) permanently displays whether a radio connection exists with one and/or with which transmitter unit (12).

3. Method according to claim 1 or 2, **characterised in that** the presence of a radio connection is no longer displayed on the transmitter unit (12) and/or receiver unit (14, 16) when a manual log-off of the respective receiver unit (14, 16) has taken place or when there is no longer a radio connection with the respective receiver unit (14, 16).

4. Method according to claim 1, 2 or 3, **characterised in that** to establish the radio connection, when at least one of the receiver units (14, 16) is within radio range of the transmitter unit (12), using its identification code (ID) the transmitter unit (12) directs a log-on request to the respective receiver unit (14, 16), and the transmitter unit (12) logs on to the respective receiver unit (14, 16) and a radio connection is established for transmission of the measurement data only when the log-on request has been confirmed at the receiver unit (14, 16).

5. Method according to claim 4, **characterised in that** the respective receiver unit (14, 16) displays whether and from which transmitter unit (12) a log-on request has been received.

6. Method according to claim 4 or 5, **characterised in that** on the basis of the log-on request, the identification code (ID) of the transmitter unit (12) is displayed at the respective receiver unit (14, 16) and that by selection and confirmation of the selection, a log-on takes place without the identification code (ID) having to be entered as such manually via in particular a keypad.

7. Method according to at least one of the preceding claims, **characterised in that** the transmitter unit (12) emits a signal when for a predefinable period there is no longer a radio connection to at least one receiver unit (14, 16).

8. Method according to at least one of the preceding claims, **characterised in that** after an interruption of the radio connection between the transmitter unit and a receiver unit which has persisted over a period of some minutes, a radio connection is automatically recreated between the transmitter unit and the receiver unit without the identification code of the transmitter unit having to be logged on again to the receiver unit.

9. Method according to claim 8, **characterised in that** a recreation of the radio connection is displayed at the transmitter unit and/or at the receiver unit.

10. Method according to claim 8 or 9, **characterised in that** with or after recreation of the radio connection, an enquiry is made as to whether the existing radio connection should be maintained or whether the existing radio connection should be interrupted.

11. Method according to at least one of the preceding claims, **characterised in that** at the transmitter unit (12) a user-defined identification word (PIN) can be allocated to the identification code (ID) of the transmitter unit (12), and/or that at the receiver unit (14, 16) a user-defined identification word (PIN) can be allocated to the identification code (ID) of the receiver unit, and used instead of the respective identification code (ID).

12. Method according to at least one of the preceding claims, **characterised in that** the transmitter unit (12) and/or the respective receiver units (14, 16) store data on when and for how long a radio connection existed with which receiver unit (14, 16) and/or transmitter unit (12).

13. Patient monitoring system (10) with a transmitter unit (12) emitting via a radio link measurement data recorded on the patient, and with multiple receiver units (14, 16) for receiving the measurement data transmitted by the transmitter unit (12), for performance of the method according to at least one of the preceding claims, wherein at the transmitter unit (12), display means (20, 34, 36) are provided for permanently displaying with which of the receiver units (14, 16) a radio connection exists.

14. System according to claim 13, **characterised in that** at the receiver unit (14, 16), display means (20, 34, 36) are provided for permanently displaying whether a radio connection exists with the transmitter unit (12).

15. System according to at least one of claims 13 or 14, **characterised in that** the transmitter unit (12) or the receiver unit (14,16) has an exchangeable battery to supply energy to the transmitter unit, and that the transmitter unit (12) or the receiver unit (14, 16) comprises an internal battery which supplies energy to the transmitter unit or the receiver unit (14, 16) respectively when the exchangeable battery is removed.

16. System according to claim 15, **characterised in that** at the receiver unit (14, 16) or transmitter unit (12), a charging station is provided for charging the exchangeable battery of the transmitter unit (12) or receiver unit (14, 16) respectively.

## Revendications

1. Procédé pour faire fonctionner un système de surveillance de patient (10) comprenant une unité émettrice (12) qui émet, via une liaison radio, des données de mesure prélevées sur un patient, et plusieurs unités de réception (14, 16) qui reçoivent les données de mesure émises par l'unité émettrice (12), un code d'identification (ID) univoque étant associé à l'unité émettrice (12) et à chacune des unités réceptrices (14, 16) et une liaison radio pour la transmission de données de mesure de l'unité émettrice (12) vers la pluralité d'unités réceptrices (14, 16) ne pouvant être établie que lorsque l'unité émettrice (12) a été connectée à l'unité réceptrice (14, 16) en utilisant son code d'identification (ID), une liaison radio transmettant des données de mesure pouvant être établie entre l'unité émettrice (12) et plusieurs unités réceptrices (14, 16), **caractérisé en ce que** s'affiche de manière durable sur l'unité émettrice (12) la ou les unités réceptrices (14, 16) avec laquelle ou lesquelles il existe une liaison radio transmettant des données de mesure.

2. Procédé selon la revendication 1, **caractérisé en ce qu'**il est affiché de manière durable non seulement sur l'unité émettrice (12) mais aussi sur l'unité réceptrice (14, 16) s'il existe une liaison radio avec une unité émettrice (12) et/ou avec quelle unité émettrice (12) il existe une liaison radio.

3. Procédé selon la revendication 1 ou la revendication 2, **caractérisé en ce que** l'existence d'une liaison radio n'est plus affichée sur l'unité émettrice (12) et/ou l'unité réceptrice (14, 16) lorsqu'une déconnexion manuelle de l'unité réceptrice considérée (14, 16) est réalisée ou lorsqu'il n'existe plus de liaison radio avec ladite unité réceptrice (14, 16).

4. Procédé selon la revendication 1, 2 ou 3, **caractérisé en ce que**, pour établir la liaison radio, lorsque l'une au moins des unités réceptrices (14, 16) se trouve dans la zone de portée radio de l'unité émettrice (12), ladite unité émettrice (12) adresse une demande de connexion à telle ou telle unité réceptrice (14, 16) en utilisant son code d'identification (ID), et la connexion de l'unité émettrice (12) à telle ou telle unité réceptrice (14, 16) ainsi que l'établissement d'une liaison radio pour la transmission des données de mesure n'ont lieu que lorsque la demande de connexion adressée à l'unité réceptrice (14, 16) est validée.

5. Procédé selon la revendication 4, **caractérisé en ce qu'**il est affiché sur l'unité réceptrice (14, 16) s'il existe une demande de connexion et en provenance de quelle unité émettrice (12).

6. Procédé selon la revendication 4 ou la revendication 5, **caractérisé en ce que**, suite à la demande de connexion, le code d'identification (ID) de l'unité émettrice (12) s'affiche sur l'unité réceptrice (14, 16) concernée et **en ce qu'**une connexion se fait par sélection et confirmation de la sélection, sans qu'il soit besoin de saisir manuellement le code d'identification (ID) en tant que tel, en particulier au moyen d'un clavier.

7. Procédé selon l'une au moins des revendications précédentes, **caractérisé en ce que** l'unité émettrice (12) émet un signal lorsqu'il n'existe plus, pendant une durée prédéfinissable, de liaison radio vers au moins une unité réceptrice (14, 16).

8. Procédé selon l'une au moins des revendications précédentes, **caractérisé en ce que** même après une interruption de quelques minutes de la liaison radio entre l'unité émettrice et une unité réceptrice, une liaison radio est automatiquement rétablie entre l'unité émettrice et l'unité réceptrice sans qu'il soit besoin d'indiquer à nouveau à l'unité réceptrice le code d'identification de l'unité émettrice.

9. Procédé selon la revendication 8, **caractérisé en ce que** le rétablissement de la liaison radio s'affiche sur l'unité émettrice et/ou l'unité réceptrice.

10. Procédé selon la revendication 8 ou la revendication 9, **caractérisé en ce que** lors de ou après le rétablissement de la liaison radio, il est demandé si la liaison radio existante doit être maintenue ou si elle doit être interrompue.

11. Procédé selon l'une au moins des revendications précédentes, **caractérisé en ce que**, sur l'unité émettrice (12), un terme d'identification défini par l'utilisateur (PIN) peut être affecté au code d'identification (ID) de l'unité émettrice (12) et/ou **en ce que**, sur l'unité réceptrice (14, 16), un terme d'identification défini par l'utilisateur (PIN) peut être affecté au code d'identification (ID) de l'unité réceptrice pour l'utiliser en lieu et place du code d'identification (ID) considéré.

12. Procédé selon l'une au moins des revendications précédentes, **caractérisé en ce que** l'on enregistre dans l'unité émettrice (12) et/ou les différentes unités réceptrices (14, 16) quand et combien de temps une liaison radio a été établie avec quelle unité réceptrice (14, 16) et/ou unité émettrice (12).

13. Système de surveillance de patient (10) comprenant une unité émettrice (12) qui émet, via une liaison radio, des données de mesure prélevées sur un patient, et plusieurs unités de réception (14, 16) qui reçoivent les données de mesure émises par l'unité émettrice, destiné à mettre en oeuvre le procédé selon l'une au moins des revendications précédentes, des moyens d'affichage (20, 34, 36) étant prévus sur l'unité émettrice (12) pour afficher de manière durable avec laquelle des unités réceptrices (14, 16) il existe une liaison radio.

14. Système selon la revendication 13, **caractérisé en ce que** des moyens d'affichage (20, 34, 36) sont prévus sur l'unité réceptrice (14, 16) pour afficher de manière durable s'il existe une liaison radio avec l'unité émettrice (12).

15. Système selon l'une au moins des revendications 13 et 14, **caractérisé en ce que** l'unité émettrice (12) et/ou l'unité réceptrice (14, 16) présentent une batterie remplaçable destinée à alimenter en énergie l'unité émettrice et **en ce que** l'unité émettrice (12) et/ou l'unité réceptrice (14, 16) comprennent une batterie interne qui alimente respectivement en énergie l'unité émettrice et/ou l'unité réceptrice (14, 16) lorsque l'on ôte la batterie remplaçable.

16. Système selon la revendication 15, **caractérisé en ce qu'**une station de charge est prévue sur l'unité réceptrice (14, 16) et/ou l'unité émettrice (12) pour charger respectivement la batterie remplaçable de l'unité émettrice (12) et/ou de l'unité réceptrice (14, 16).
